Europäisches Patentamt

European Patent Office

Office européen des brevets

(19)

(11) Numéro de publication : **0 187 600**
**B1**

(12) **FASCICULE DE BREVET EUROPÉEN**

(45) Date de publication du fascicule du brevet :
25.05.88

(21) Numéro de dépôt : 85402631.7

(22) Date de dépôt : 24.12.85

(51) Int. Cl.⁴ : **C 07 C   9/04, C 07 C   1/04,
B 01 J 23/28, B 01 J 23/88**

(54) Procédé de production du méthane à l'aide d'un catalyseur thiorésistant et catalyseur pour la mise en oeuvre de ce procédé.

(30) Priorité : 28.12.84 FR 8420069

(43) Date de publication de la demande :
16.07.86 Bulletin 86/29

(45) Mention de la délivrance du brevet :
25.05.88 Bulletin 88/21

(84) Etats contractants désignés :
AT BE CH DE GB IT LI LU NL SE

(56) Documents cités :
US-A- 4 151 191
Le dossier contient des informations techniques
présentées postérieurement au dépôt de la demande
et ne figurant pas dans le présent fascicule.

(73) Titulaire : GAZ DE FRANCE
23, rue Philibert Delorme
F-75017 Paris (FR)

(72) Inventeur : Przydrozny, Michel
32 rue André Musager
F-95630 Mériel (FR)
Inventeur : D'Emmerez de Charmoy, Roger Dennisson
7 Impasse St. Maur
F-94700 Maisons-Alfort (FR)
Inventeur : Sauvion, Guy Noel
8 rue du Béarn
F-94550 Chevilly-Larue (FR)
Inventeur : Caillod, Jack
31 rue Phanie Leleu
F-95150 Taverny (FR)

(74) Mandataire : Durand, Yves Armand Louis et al
Cabinet Z. Weinstein 20, Avenue de Friedland
F-75008 Paris (FR)

## Description

La présente invention a essentiellement pour objet un procédé de production du méthane ou d'un mélange de gaz comprenant du méthane à l'aide d'un catalyseur résistant au soufre au thiorésistant.

Elle vise également le catalyseur pour la mise en œuvre de ce procédé.

Il est déjà connu de réaliser la synthèse du méthane à partir d'un mélange de gaz comprenant entre autres du monoxyde de carbone, de l'hydrogène, de l'eau et des composés soufrés, ledit mélange de gaz pouvant en particulier être obtenu par gazéification du charbon et étant mis en contact avec un catalyseur susceptible d'activer la réaction de synthèse.

Dans ce but, divers catalyseurs ont été proposés, mais on a constaté qu'ils s'usent ou se désactivent rapidement en raison de la présence des composés soufrés.

C'est ainsi que l'on a déjà proposé des catalyseurs pour la réaction de méthanisation qui sont à base par exemple de molybdène et/ou de nickel déposés sur un support d'alumine, mais ces catalyseurs sont loin d'être très satisfaisants sur le plan de leur activité et de leur sélectivité en faveur de la production de méthane.

On connaît également d'après le document US-A-4.151.191 un procédé de production du méthane qui consiste à mettre en contact un mélange de monoxyde de carbone, d'hydrogène et de composés soufrés avec un catalyseur massique obtenu par co-précipitation et comprenant par exemple et entre autes du molybdène et du cérium, mais un tel catalyseur possède une structure qui ne permet pas d'obtenir des résultats satisfaisants sur le plan de la conversion du CO et de la sélectivité en $CH_4$.

Aussi, la présente invention a pour but de remédier à ces inconvénients en proposant un procédé de production du méthane à l'aide d'un catalyseur thiorésistant qui donnent d'excellents résultats tant sur le plan de la conversion du monoxyde de carbone que sur celui de la sélectivité en méthane produit et de la stabilité des performances du catalyseur au cours du temps.

A cet effet, l'invention a pour objet un procédé de production du méthane ou d'un mélange comprenant du méthane, à partir d'un mélange de départ comprenant notamment du monoxyde de carbone, de l'hydrogène, et des composés soufrés, et dans lequel on met en contact ledit mélange de départ avec un catalyseur thiorésistant à une température comprise entre 250 et 650 °C et à une pression comprise entre 5 et 140 bars, caractérisé en ce que ledit catalyseur comprend un métal choisi parmi le molybdène, le vanadium ou le tungstène et éventuellement un promoteur d'activité constitué par du cobalt et/ou du nickel lequel catalyseur est déposé sur un support d'oxyde de cérium.

Comme on le verra plus loin, la sélectivité en méthane produit par ce procédé est très supérieure à celle obtenue avec les procédés antérieurs, en raison du fait essentiel que l'on utilise, selon la présente invention, un catalyseur déposé sur oxyde de cérium.

Suivant une autre caractéristique du procédé selon l'invention, la réaction est effectuée à une vitesse spatiale (V.V.h.) comprise entre 100 et 15 000 heures $^{-1}$ et pour un rapport molaire hydrogène/monoxyde de carbone égal à au moins 0,3 et de préférence égal à 1.

Suivant un mode de réalisation préféré, la réaction est effectuée à une vitesse spatiale égale à 4 750 heures $^{-1}$ et pour un rapport molaire hydrogène/monoxyde de carbone égal à 1, à une pression égale à 30 bars et à une température comprise entre 300 et 600 °C et de préférence égale à 500 °C.

L'invention vise également un catalyseur pour la mise en œuvre du procédé répondant à l'une ou l'autre des caractéristiques ci-dessus, ce catalyseur étant caractérisé en ce qu'il est représenté par la formule $X/CeO_2$, $X/Co/CeO_2$, $X/Ni/CeO_2$ ou $X/Co/Ni/CeO_2$ dans laquelle X est le molybdène, le vanadium ou le tungstène et en ce qu'il est défini par les caractéristiques suivantes :

— surface spécifique BET du support de $CeO_2$ supérieure à 10 $m^2/g$,

— volume poreux total compris entre 0,15 et 0,5 $cm^3/g$,

— densité de remplissage tassé comprise entre 0,5 et 2,5,

— rapport atomique du métal X précité au cérium compris entre 1/50 et 1/4, et

— rapport atomique du métal promoteur d'activité (cobalt et/ou nickel) ou métal X compris entre 0 et 1.

Selon un mode de réalisation préféré, la surface spécifique BET du catalyseur ci-dessus est égale à 50 $m^2/g$, son volume poreux total est compris entre 0,3 et 0,4 $cm^3/g$, la densité de remplissage tassé est comprise entre entre 1 et 2, le rapport atomique X/cérium est compris entre 1/20 et 1/7, et le rapport atomique métal promoteur/X est compris entre 0,1 et 0,5.

Ce catalyseur peut être préparé par les techniques courantes et en particulier par imprégnation du support par des solutions des précurseurs des métaux que l'on désire introduire.

Mais d'autres caractéristiques et avantages de l'invention apparaîtront mieux dans l'exemple ci-après qui ne doit pas être interprété comme limitant la présente invention.

On a tout d'abord préparé trois catalyseurs déposés sur oxyde de cérium, et à savoir : un catalyseur molybdène/oxyde de cérium, un catalyseur cobalt/molybdène/oxyde de cérium, et un catalyseur nickel/molybdène/oxyde de cérium.

Le catalyseur $Mo/CeO_2$ présentait une surface spécifique BET de 42 $m^2/g$, un volume poreux de 0,14 $cm^3/g$, une densité de remplissage tassé de 1,83, et une teneur en molybdène de 3,6 % en poids.

Le catalyseur $Co/Mo/CeO_2$ présentait une surface spécifique BET de 39 $m^2/g$, un volume poreux à

2

l'eau de 0,11 cm³/g et une densité de remplissage tassé de 1,92.

Enfin, le catalyseur Ni/Mo/CeO² présentait une surface spécifique BET de 37 m²/g, un volume poreux à l'eau de 0,11 cm³/g et une densité de remplissage tassé de 1,95.

Ensuite, on a procédé à un test catalytique pour comparer les performances des trois catalyseurs ci-dessus avec des catalyseurs connus à base d'alumine ou d'aluminium et répondant aux formules suivantes : $Mo/Al_2O_3$, $Co/Mo/Al_2O_3$, $Ni/Mo/Al_2O_3$ et $Ce/Mo/Al$.

Ce test catalytique, qui avait pour but de comparer les performances de tous les catalyseurs ci-dessus en termes d'activité et de sélectivité dans la préparation du méthane, a consisté à mettre en contact tous ces catalyseurs avec un mélange de réactifs comprenant dans un premier temps (conditions du tableau 1) 37,25 % en volume de CO (monoxyde de carbone), 37,25 % en volume de $H_2$, 25 % en volume de $H_2O$ et 0,5 % en volume de $H_2S$ et dans un deuxième temps (conditions du tableau 2) 49,75 % en volume de $H_2$ et 0,5 % en volume de $H_2S$.

La réaction a été effectuée à une vitesse spatiale de 4 750 heures $^{-1}$, sous une pression de 30 bars et à une température de 500 °C, après que les catalyseurs aient subi un traitement de présulfuration à 350 °C pendant 6 heures sous un courant de 10 litres/h d'un mélange de 1,3 % de $H_2S$ dans l'hydrogène.

Après séparation de l'eau par un condenseur, on a analysé les gaz de sortie par chromatographie en phase gazeuse, ce qui a permis de doser notamment CO, $CO_2$, $CH_4$, $C_2H_6$ et $C_3H_8$.

Cette analyse a permis de calculer d'une part le taux de conversion du monoxyde de carbone (%), défini par le rapport :

$$t_{CO} = \frac{\text{moles de CO consommées}}{\text{moles de CO introduites}} \times 100$$

et d'autre part, la sélectivité en $CH_4$ (%), définie par le rapport :

$$S_{CH4} = \frac{\text{moles de CH}_4 \text{ formées}}{\text{moles de CO consommées}} \times 100$$

Les tableaux 1 et 2 ci-après illustrent les résultats obtenus sur tous les catalyseurs décrits précédemment, avant et après un vieillissement correspondant à 20 heures de fonctionnement ou de travail du catalyseur.

(Voir Tableau 1 page 4)
(Voir Tableau 2 page 5)

3

Tabelle 1

| Catalyseurs | | Avant vieillissement | | après vieillissement | |
|---|---|---|---|---|---|
| | | $t_{CO}$ % | $S_{CH_4}$ % | $t_{CO}$ % | $S_{CH_4}$ % |
| Art antérieur | $Mo/Al_2O_3$ | 61,7 | 15,2 | 57,4 | 12,1 |
| | $Co/Mo/Al_2O_3$ | 62,6 | 24,8 | 61,6 | 19,1 |
| | $Ni/Mo/Al_2O_3$ | 66,7 | 22,3 | 65 | 18,5 |
| | $Ce/Mo/Al$ | 71 | 21,9 | 64,8 | 20,8 |
| Invention | $Mo/CeO_2$ | 80,4 | 30,4 | 77 | 28 |
| | $Co/Mo/CeO_2$ | 87,3 | 36,7 | 81,1 | 32,7 |
| | $Ni/Mo/CeO_2$ | 88,1 | 35,3 | 81,5 | 31,9 |

0 187 600

Tabelle 2

| | Catalyseurs | Avant vieillissement | | après vieillissement | |
|---|---|---|---|---|---|
| | | $t_{CO}$ % | $S_{CH_4}$ % | $t_{CO}$ % | $S_{CH_4}$ % |
| Art antérieur | Mo/Al$_2$O$_3$ | 78,3 | 49,9 | 69,4 | 50,0 |
| | Co/Mo/Al$_2$O$_3$ | 79,1 | 50,0 | 70,9 | 49,5 |
| | Ni/Mo/Al$_2$O$_3$ | 78,8 | 49,6 | 71,9 | 49,8 |
| Invention | Mo/CeO$_2$ | 85,8 | 49,7 | 78,2 | 49,8 |
| | Co/Mo/CeO$_2$ | 87,1 | 50,0 | 78,7 | 50,0 |
| | Ni/Mo/CeO$_2$ | 86,6 | 48,0 | 79,0 | 47,3 |

0 187 600

**0 187 600**

Les résultats donnés dans le tableau 1 mettent clairement en évidence la supériorité des catalyseurs selon l'invention par rapport aux catalyseurs de l'art antérieur pour ce qui est du taux de conversion du monoxyde de carbone, de la sélectivité en méthane produit, et de la stabilité des performances au cours du temps.

Plus précisément, on remarquera que le catalyseur $Mo/CeO_2$ est non seulement supérieur aux trois premiers catalyseurs de l'art antérieur ne contenant pas d'oxyde de cérium, mais est également supérieur au quatrième catalyseur de l'art antérieur mentionné dans le tableau 1 et qui est à base de cérium, de molybdène et d'aluminium.

Cette supériorité est également clairement visible en ce qui concerne les catalyseurs $Co/Mo/CeO_2$ et $Ni/Mo/CeO_2$, si on les compare aux catalyseurs de l'art antérieur et notamment aux catalyseurs de Co ou Ni avec molybdène déposés sur alumine. On voit en effet ici qu'on obtient avec ces deux catalyseurs une sélectivité en méthane comprise entre 32 et 36 %, et des taux de conversion en CO supérieurs à 80 %.

Les résultats donnés dans le tableau 2 (absence d'eau dans le mélange de départ) montrent que l'on obtient des taux de conversion en CO supérieurs à 85 % et nettement meilleurs que ceux obtenus avec les catalyseurs de l'art antérieur. Les résultats sont également meilleurs pour ce qui est de la stabilité des performances au cours du temps.

Mais ici, la sélectivité en méthane produit est sensiblement la même que celle des catalyseurs de l'art antérieur et se situe dans tous les cas au niveau de la limite thermodynamique.

On a donc réalisé suivant l'invention un procédé de méthanisation qui permet de récupérer des quantités importantes de méthane en utilisant un catalyseur très stable à base d'oxyde de cérium et résistant efficacement aux composés soufrés tels que $H_2S$, COS, $CS_2CH_3S$ etc... et dont la teneur peut dépasser 4 % molaire en soufre.

Bien entendu, l'invention n'est nullement limitée au mode de réalisation ou à l'exemple décrit ci-dessus.

C'est ainsi qu'en remplaçant le molybdène par du vanadium ou du tungstène dans les formules de catalyseurs conformés au principe de l'invention, on obtient des résultats et des performances proches de ceux décrits ci-dessus. Egalement, l'utilisation conjointe de deux métaux promoteurs d'activité (cobalt et nickel), au lieu d'un seul, conduirait à un catalyseur tout aussi actif en faveur de la production de méthane.

**Revendications**

1. Procédé de production du méthane ou d'un mélange de gaz comprenant du méthane à partir d'un mélange de départ comprenant notamment du monoxyde de carbone, de l'hydrogène, et des composés soufrés, et dans lequel on met en contact ledit mélange de départ avec un catalyseur thiorésistant à une température comprise entre 250 et 650 °C et à une pression comprise entre 5 et 140 bars, caractérisé en ce que ledit catalyseur comprend un métal choisi parmi le molybdène, le vanadium ou le tungstène et éventuellement un promoteur d'activité constitué par du cobalt et/ou du nickel, lequel catalyseur est déposé sur un support d'oxyde de cérium.

2. Procédé selon la revendication 1, caractérisé en ce que la réaction est effectuée à une vitesse spatiale comprise entre 100 et 15 000 heures $^{-1}$ et pour un rapport molaire hydrogène/monoxyde de carbone égal à au moins 0,3 et de préférence égal à 1.

3. Procédé selon la revendication 1 ou 2, caractérisé en ce que la réaction est effectuée à une vitesse spatiale égale à 4 750 heures $^{-1}$ et pour un rapport molaire hydrogène/monoxyde de carbone égal à 1, à une pression égale à 30 bars et à une températrue comprise entre 300 et 600 °C et de préférence égale à 500 °C.

4. Catalyseur pour la mise en œuvre du procédé selon l'une des revendications 1 à 3, caractérisé en ce qu'il répond à la formule $X/CeO_2$, $X/Co/CeO_2$ ou $X/Ni/CeO_2$ ou $X/Co/Ni/CeO_2$ dans laquelle X est le molybdène, le vanadium ou le tungstène et en ce que ledit catalyseur est défini par les caractéristiques suivantes :
— surfaces spécifiques BET du rapport de $CeO_2$ supérieure à 10 $m^2/g$ ;
— volume poreux total compris entre 0,15 et 0,5 $cm^3/g$ ;
— densité de remplissage tassé comprise entre 0,5 et 2,5 ;
— rapport atomique du métal X précité au cérium compris entre 1/50 et 1/4, et
— rapport atomique du métal promoteur d'activité (Co et/ou Ni) au métal X compris entre 0 et 1.

5. Catalyseur selon la revendication 4, caractérisé en ce que la surface spécifique BET précitée est égale à 50 $m^2/g$, le volume poreux total précité est compris entre 0,3 et 0,4 $cm^3/g$, la densité de remplissage tassé est comprise entre 1 et 2, le rapport atomique X/cérium est compris entre 1/20 et 1/7, et le rapport atomique métal promoteur/X est compris entre 0,1 et 0,5.

**Claims**

1. Process for the preparation of methane or of a gas mixture comprising methane from a starting

6

mixture comprising in particular carbon monoxide, hydrogen and sulphur compounds, and in which the said starting mixture is contacted with a thioresistant catalyst, at a temperature comprised between 250 and 650 ºC and under a pressure comprised between 5 and 140 bars, characterised in that the said catalyst comprises a metal selected from molybdenum, vanadium or tungsten and if necessary an activity promoter consisting of cobalt and/or nickel, said catalyst being deposited on a support of cerium oxide.

2. Process according to claim 1, characterized in that the reaction is carried out at space velocity comprised between 100 and 15 000 hours $^{-1}$ and for a hydrogen/carbon monoxide molar ratio at least equal to 0.3 and preferably equal to 1.

3. Process according to claim 1 or 2, characterized in that the reaction is carried out at a space velocity equal to 4 750 hours $^{-1}$ and for a hydrogen/carbon monoxide molar ration equal to 1, under a pressure equal to 30 bars and at a temperature comprised between 300 and 600 ºC and preferably equal to 500 ºC.

4. Catalyst for carrying out the process according to one of claims 1 to 3, characterized in that it corresponds to the formula $X/CeO_2$, $X/Co/CeO_2$ or $X/Ni/CeO_2$ or $X/Co/Ni/CeO_2$ where X is molybdenum, vanadium or tungsten and in that the said catalyst is defined by the following characteristics :

BET specific surface area of the support of $CeO_2$ higher than 10 $m^2/g$ ;

total pore volume comprised between 0.15 and 0.5 $cm^3/g$ ;

packed filling density comprised between 0.5 and 2.5 ;

atomic ratio of the aforesaid metal X to cerium comprised between 1/50 and 1/4, and

atomic ration of the metallic activity promoter (Co and/or Ni) to the metal X comprised between 0 and 1.

5. Catalyst according to claim 4, characterized in that the aforesaid BET specific surface area is equel to 50 $m^2/g$, the aforesaid total pore volume is comprised between 0.3 and 0.4 $cm^3/g$, the packed filling density is comprised between 1 and 2, the X/cerium atomic ration is comprised between 1/20 and 1/7, and the metallic promoter/X atomic ratio is comprised between 0.1 and 0.5.

**Patentansprüche**

1. Verfahren zur Herstellung von Methan oder einer methanhaltigen Gasmischung aus einer Ausgangsmischung, die insbesondere Kohlenmonoxyd, Wasserstoff und Schwefelverbindungen enthält, und wobei die besagte Ausgangsmischung mit einem schwefelwiderstandsfähigen Katalysator, bei einer zwischen 250 und 650 ºC liegenden Temperatur und unter einem zwischen 5 und 140 Bar liegenden Druck, in Kontakt, gebracht ist, dadurch gekennzeichnet, daß der besagte Katalysator ein, unter Molybdän, Vanadium oder Wolfram gewähltes Metall und gegebenenfalls einen Wirksamkeitspromotor aufweist, der aus Kobalt und/oder Nickel besteht, wobei der besagte Katalysator auf einem Träger aus Ceriumoxyd abgesetzt ist.

2. Verfahren nach Anspruch 1, dadurch gekennzeichnet, daß die Reaktion bei einer Raumgeschwindigkeit zwischen 100 und 15 000 Stunden $^{-1}$ und für ein Wasserstoff/Kohlenmonoxydmolverhältnis von wenigstens 0,3 und vorzugsweise von 1 durchgeführt ist.

3. Verfahren gemäß Anspruch 1 oder 2, dadurch gekennzeichnet, daß die Reaktion bei einer Raumgeschwindigkeit von 4 750 Stunden $^{-1}$ and für ein Wasserstoff/Kohlenmonoxydmolverhältnis von 1, unter einem Druck von 30 Bar und bei einer temperatur zwischen 300 und 600 ºC, und vorzugsweise von 500 ºC, durchgeführt ist.

4. Katalysator zur Durchführung des Verfahrens gemäß einem der Anspruche 1 bis 3, dadurch gekennzeichnet, daß der Formel $X/CeO_2$, $X/Co/CeO_2$ oder $X/Ni/CeO_2$ oder $X/Co/Ni/CeO_2$ entspricht, in welcher X für Molybdän, Vanadium oder Wolfram steht, und daß der besagte Katalysator durch die folgenden Merkmale bestimmt ist :

— BET Oberflächenkennzahl des Trägers aus $CeO_2$ größer als 10 $m^2/g$ ;

— Gesamtporenvolumen zwischen 0,15 et 0,5 $cm^3/g$ ;

— Kompaktfüllungsdichte zwischen 0,5 und 2,5 ;

— Atomverhältnis der vorerwähnten Metalls X zu Cerium zwischen 1/50 und 1/4, und

— Atomverhältnis des metallischen Wirksamkeitspromotors (CO und/oder Ni) zum Metall X zwischen 0 und 1.

5. Katalysator gemäß Anspruch 4, dadurch gekennzeichnet, daß die vorerwähnte BET Oberflächenkennzahl 50 $m^2/g$ beträgt, das vorerwähnte Gesamtporenvolumem zwischen 0,3 und 0,4 $cm^3/g$ liegt, die Kompaktfüllungsdichte zwischen 1 und 2 liegt, das Atomverhältnis X/Cerium zwischen 1/20 und 1/7 liegt, und das Atomverhältnis metallischer Promotor/X zwischen 0,1 und 0,5 liegt.